# EUROPEAN PATENT SPECIFICATION

(11) **EP 2 227 185 B1**
(45) Date of publication and mention of the grant of the patent: **01.05.2013**
(21) Application number: 08848004.1
(22) Date of filing: 06.11.2008
(51) Int. Cl.: A61F 2/966

(54) **APPARATUS FOR INTRODUCING EXPANDABLE INTRALUMINAL PROSTHESIS**
GERÄT ZUR EINFÜHRUNG VON EXPANDIERBAREN INTRALUMINALEN PROTHESEN
APPAREIL PERMETTANT L'INSERTION D'UNE PROTHÈSE INTRALUMINALE EXTENSIBLE

(30) Priority: 07.11.2007 US 2200 P
(43) Date of publication of application: 15.09.2010
(73) Proprietor: Cook Medical Technologies LLC, Bloomington, IN 47404 (US)
(72) Inventor: HANSEN, Palle, M., DK 4632 Bjaeverskov (DK)
(74) Representative: Garratt, Peter Douglas
(86) International application number: PCT/US2008/082580
(87) International publication number: WO 2009/061882

(56) References cited:
- EP-A- 0 732 087
- EP-A- 1 314 447
- WO-A-03/039345
- WO-A-2006/015323
- WO-A-2008/121380
- US-B1- 6 254 628

## Description

### Technical Field

The present invention relates to medical devices and in particular to an apparatus for introducing an expandable prosthesis into body lumens.

### Background of the Invention

A wide variety of techniques exist for introducing self-expandable prostheses into body lumens. One such technique involves a surgeon advancing a guide-wire through body lumens so that the wire extends through the passage in which it is desired to implant the self-expandable prosthesis, such as a stent. An introducer is then passed over the guide-wire from outside the patient to deliver the stent to the desired location.

A typical introducer comprises a catheter having a wire guide lumen and an atraumatic tip at its distal end. The stent is positioned just proximally of the distal tip of the catheter. An outer sheath extends over the exterior surfaces of the catheter and the stent, so as to restrain the stent during introduction. This introducer is advanced to the deployment location within a body lumen, whereupon the surgeon retracts the outer sheath relative to the catheter so as to gradually unsheathe the self-expanding stent and allow it to engage the vessel wall.

Retraction of an outer sheath which extends the length of the catheter can present problems. The length of sheath that has to be moved and the frictional resistance to movement can lead to uncertainties in the positioning of the stent. There can also be an increased risk of damage to the intimal cells. For this reason, it has been proposed in prior art constructions to reduce the length of the outer sheath; a shortened outer sheath is located near the distal end of the introducer covering only the stent and - usually - a small portion of the distal end of the catheter. Typically, a pull wire is attached to the shortened outer sheath to enable it to be retracted remotely.

If a pull wire becomes twisted circumferentially around the catheter, there is not only an increased frictional resistance, but also the risk of unwanted torque being applied to the outer sheath. It has for this reason been suggested to locate the pull wire in a lumen within the catheter. It is necessary, however, to accommodate retraction of the sheath and for this reason the pull wire lumen is replaced by an open channel or groove at the distal end of the catheter, over a length at least equal to the length of the outer sheath.

There remain difficulties with such an arrangement. That portion of the pull wire that lies within the open channel or groove can become dislodged and prone to twisting. This risk will increase with the length of the channel or groove and it is therefore desirable for the length of the channel or groove to match that of the sheath. This however creates manufacturing and inventory problems since a different catheter must be designed for each sheath length.

Reference is directed to WO 03/039345, US 6 254 628 and EP 0 732 087 each of which disclose a sheath which can be torn by remote retraction of a pull wire to deliver a device held within the tearable sheath.

### Summary of the Invention

Thus, there is provided in accordance with one aspect of the present invention apparatus for delivering an expandable prosthesis within a body lumen, comprising a catheter structure having at its distal end a prosthesis bearing site; a delivery sheath positioned over said prosthesis bearing site so as to retain a prosthesis as the catheter structure is advanced through the body lumen, the delivery sheath being retractable to release the prosthesis; a pull wire lumen extending at least part way through the catheter structure from the proximal end of the delivery sheath; and a pull wire connected with the delivery sheath and extending through the pull wire lumen to enable remote retraction of the delivery sheath, wherein the distal end of the pull wire lumen is associated with a region of weakness in the catheter structure such that the region of weakness is torn on retraction of the delivery sheath.

The pull wire lumen can extend directly to the sheath with no need for an open channel or groove to accommodate retraction of the sheath. During introduction of the prosthesis to the delivery site, the entire length of the or each pull wire can accordingly remain within the corresponding pull wire lumen, with no risk of circumferential twisting. One catheter may be designed for combination with a range of delivery sheaths of varying lengths, the different retracting lengths being accommodated by tearing of different lengths of the region of weakness associated with the pull wire lumen.

The exterior surface of the catheter structure, free of grooves, may assist in advancement of the catheter structure. The reaction of the catheter structure to initial proximal retraction of the delivery sheath - before sufficient force is applied to tear the region of weakness - may assist in resisting early or inadvertent retraction of the delivery sheath.

The apparatus may further comprise a barb that, on retraction of the delivery sheath, aids in tearing said region of weakness, the barb being formed on an annular member located around said catheter structure or on the delivery sheath. There may be a plurality of pull wires and optionally also of such barbs spaced equiangularly about said catheter structure.

The regions of weakness may be provided at least in part by grooves formed in said catheter structure. An inner sheath may consist substantially of a first material with the regions of weakness being provided at least in part by elongate regions of a second material having optionally lower shear strength or lower scratch hardness than said first material. The second material may be anisotropic.

It is here described a method for delivering an expandable prosthesis within body lumens, said method comprising the steps of positioning in a body lumen an apparatus having a longitudinal axis, said apparatus comprising a catheter structure having at its distal end a prosthesis bearing site; an expandable prosthesis located at said prosthesis bearing site; a delivery sheath positioned over said prosthesis bearing site so as to retain the prosthesis as the catheter structure is advanced through the body lumen, the delivery sheath being retractable to release the prosthesis; a pull wire lumen extending at least part way through the catheter structure from the proximal end of the delivery sheath; and a pull wire connected with the delivery sheath and extending through the pull wire lumen to enable remote retraction of the delivery sheath, wherein the distal end of the pull wire lumen is associated with a region of weakness in the catheter structure; and pulling said pull wire proximally along said axis so as to retract said delivery sheath, thus unsheathing said prosthesis, and tearing said region of weakness.

Further objects, features and advantages of this invention will become readily apparent to person skilled in the art after a review of the following description, with reference to the drawings and claims that are appended to and form a part of this specification.

### Description of the Drawings

Figure 1 is an axial section through the distal region of an introducer in accordance with a first embodiment of the present invention
Figure 2 is a partial isometric view of the distal end of the inner sheath of the first embodiment before retraction of the outer delivery sheath.
Figure 3 is a view similar to that of Figure 2 following partial retraction of the outer sheath.
Figure 4 is a radial cross sectional view of an introducer according to a further embodiment of the present invention.
Figure 5 is a radial cross sectional view of an introducer according to a still further embodiment of the present invention.
Figure 6 is a radial cross sectional view of yet a further embodiment of the present invention having an annular cutting member.
Figure 7 is an axial cross sectional view of the introducer depicted in Figure 1 within a body lumen before deployment of a self-expanding stent.
Figure 8 is a view similar to that of Figure 7 with the self-expanding stent partially deployed.
Figure 9 is a view similar to that of Figures 7 and 8 with the self-expanding stent fully deployed.

### Detailed Description of the Invention

The example will be taken of apparatus for delivering an expandable prosthesis within body lumens in the form of an introducer for delivering a self-expandable stent.

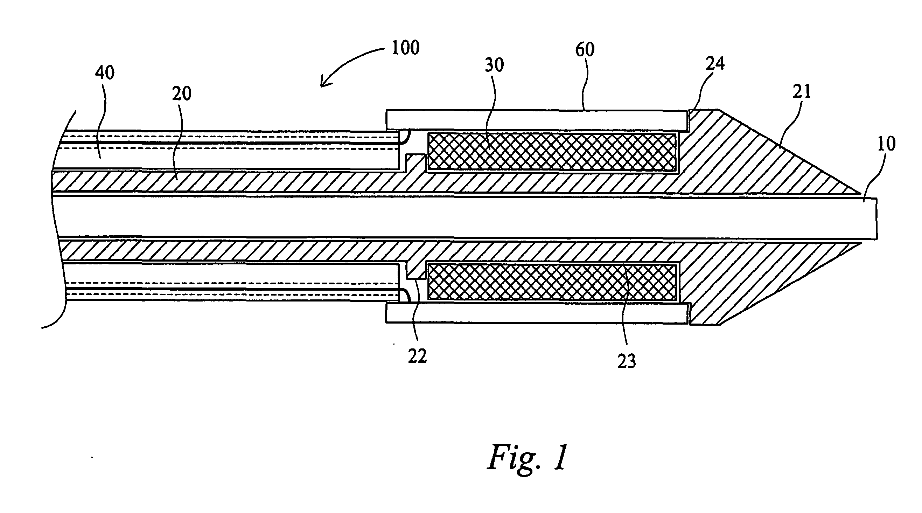

Figure 1 shows an introducer (100) comprising a catheter (20) configured for introduction over a guide wire (10). The catheter (20) provides at its distal end a bearing site (23) for a stent (30), the bearing site being defined between an atraumatic distal tip (21) and a stent pusher ring (22). The atraumatic distal tip (21) and the stent pusher ring (22) may be formed integrally in the catheter (20) or may - through a variety of techniques well known in the art - be formed from separate components bonded or appropriately secured to the core catheter. The atraumatic distal tip (21) may have a substantially frustoconical shape.

An inner sheath (40) extends coaxially over the catheter (20) from the proximal end of the catheter to the stent pusher ring (22). This inner sheath (40) will typically be of a different material than that of the catheter (20) and may be appropriately bonded or secured to the catheter so as to be fixed against movement relative to the catheter (20) whether axial or circumferential. An outer or delivery sheath (60), which is very much shorter than the catheter (20) and the inner sheath (40) and which may be only just longer than the stent (30), is disposed over the stent (30) preferably covering the distal extremity of the inner sheath (40). The distal tip (21) of the catheter is preferably provided with an annular recess (24) to accommodate the distal end of the outer sheath. As will subsequently be described in more detail, the outer or delivery sheath (60) is retractable proximally relative to the catheter to release the stent.

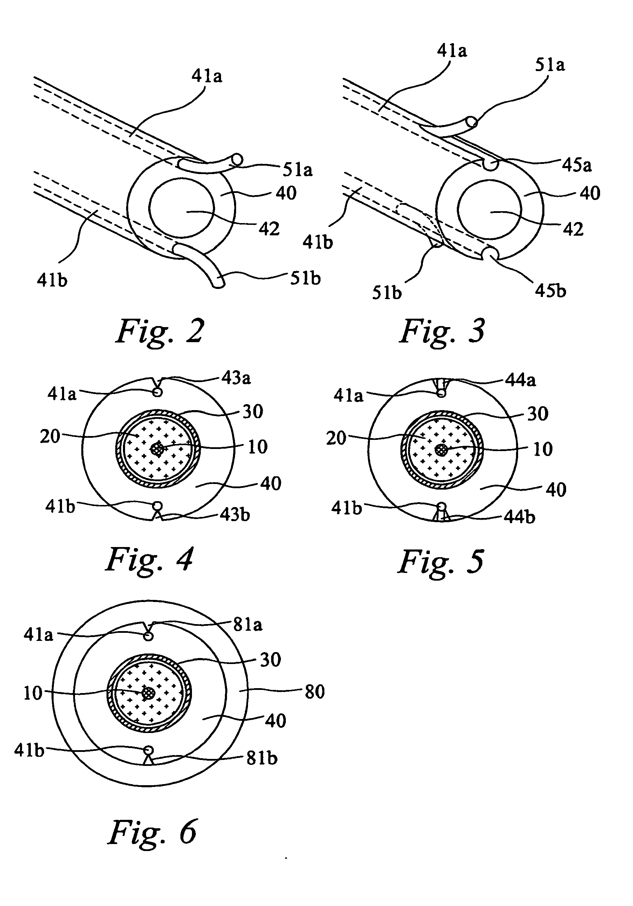

The inner sheath (40) will now be described in more detail, making reference also to Figures 2 and 3.

The inner sheath (40) has a main lumen (42) which extends along its longitudinal axis so as to make the inner sheath (40) substantially tubular. The inner sheath also comprises two pulling wire lumens (41 a, 41 b) which are disposed either side of the central lumen (42) and extend parallel to the longitudinal axis. These are equiangularly spaced about the longitudinal axis. Disposed within each pulling wire lumen (41 a, 41 b) is one of two pulling wires (50a, 50b) which extend along the length of the introducer and may be operated by the surgeon from exterior the patient. The pulling wires (50a, 50b) emerge from the distal end of the inner sheath through the distal ends of the pulling wire lumens (41 a, 41 b) and are attached to the interior of the outer sheath (60). The inner sheath (40) may comprise any suitable biocompatible material such as polytetrafluoroethylene (PFTE), polyetheretherketones (PEEK), polyvinyl chloride (PVC), polyimide, polyimide reinforced with a stainless steel braid (provided interior the pulling wire lumens 41a, 41 b), polyurethane and the like. The exterior of inner sheath (40) may be provided with a lubricous coating so as to reduce friction between the outer surface and the body lumens during introduction and to reduce friction between the outer sheath and inner sheath during retraction of the outer sheath. It will be noted that in this configuration, the exterior surface of the inner sheath is smooth and continuous.

Figure 3 displays the inner sheath (40), with the pulling wires (50a, 50b) partially retracted so as to pull back the outer sheath (not shown in Figure 3) and partially unsheathe the stent (not shown in Figure 3) which is located adjacent the distal end of the inner sheath (40). The pulling wire lumens (41 a, 41 b) are situated sufficiently close to the surface of inner sheath (40) that when the pulling wires (50a, 50b) are retracted the distal ends of the pulling wires (51 a, 51 b) tear slits (45a, 45b) in the radially exterior surface of the inner sheath (40). Thus, the outer sheath (60) may only be retracted by tearing of the material of inner sheath (40) and specifically by the tearing of a region of weakness comprising a strip region of the inner sheath bounding the associated pull wire lumen to the radially outward side. The weakness arises in this case from the small radial dimension of the strip region and from the material chosen for the inner sheath. Retraction of the outer sheath (60) may only be accomplished by a conscious act of the surgeon and accidental retraction is substantially prevented. For this purpose, the inner sheath (40) may comprise a soft biocompatible polymeric material to encourage tearing of the slits (45a, 45b). Suitably, the inner sheath (40) may comprise longitudinally molecularly orientated, anisotropic material such as PFTE whose molecular properties permit it to be torn longitudinally along slit lines adjacent the pulling wire lumens (41.a, 41 b). The use of two (or more) equiangularly spaced pull wires may assist in ensuring that the retracting movement of the delivery sheath lies wholly on the longitudinal axis without rocking or twisting.

Figure 4 displays a cross section through an introducer according to further embodiment of the present invention, in which two elongate regions of weakness that extend longitudinally along the radially exterior surface of inner sheath (40) include grooves (43a, 43b) cut into the surface of inner sheath (40) adjacent the pulling wire lumens (41 a, 41 b). The grooves serve to reduce the amount of material that the distal ends of the pulling wires (51 a, 51 b) are required to tear through. Also shown is the guide wire (10) running along the longitudinal axis of the central lumen (42) and catheter (20) extending over the length of guide wire (10).

Figure 5 displays a cross section through a still further embodiment of the present invention, where elongate regions of weakness are formed in inner sheath (40) by providing elongate regions of frangible material (44a, 44b). These portions of frangible material are formed adjacent the pulling wire lumens (41 a, 41 b) so that distal tips of the pulling wires (51 a, 51 b) need only tear through the frangible material. The portions of frangible material (44a, 44b) are suitably manufactured in a biocompatible polymer with less resistance to tearing than the material of inner sheath (40). In particular, the frangible material may have a lower shear strength and/or scratch hardness than the material of the remainder of the sheath so that there is a substantial contrast in the resistance to tearing at the boundary of the regions of frangible material (44a, 44b). Further, the regions of frangible material (44a, 44b) may consist of longitudinally molecularly orientated, anisotropic material such as PFTE whose molecular properties permit it to be torn longitudinally along slit lines adjacent the pulling wire lumens (41 a, 41 b), whilst the remainder of the inner sheath consists of ordinary PFTE or a similar biocompatible polymer.

Figure 6 displays a cross sectional view through an introducer according to a still further embodiment of the present invention. This embodiment is similar to that pictured in Figure 4 with the addition of an annular cutting member (80) which encircles the radially exterior surface of inner sheath (40). The annular member has two opposing and radially in-facing barbs (81 a, 81 b), which in use pierce the surface of interior sheath (40) so that proximal movement of the annular member scores slits in the surface of inner sheath (40). Thus, the annular member facilitates proximal movement of the ends of the pulling wires (51a, 51b). In this embodiment, the tips of the barbs (81a, 81b) are of sufficient depth to reach the guide wire lumens (41 a, 41 b), but it will be appreciated that the barbs may penetrate to various depths in other embodiments of the present invention. The outer sheath (not shown in Figure 6) is disposed distally to the annular cutting member and urges the annular cutting member proximally by contact between their opposing end surfaces. In some embodiments the annular cutting member (80) may be formed integrally with the outer sheath, so that in effect an outer sheath is provided with two radially in-facing barbs. The point of attachment of the distal ends of the pulling wires (51 a, 51 b) to the outer sheath (not shown) may be distal to the locations of the barbs (81 a, 81 b).

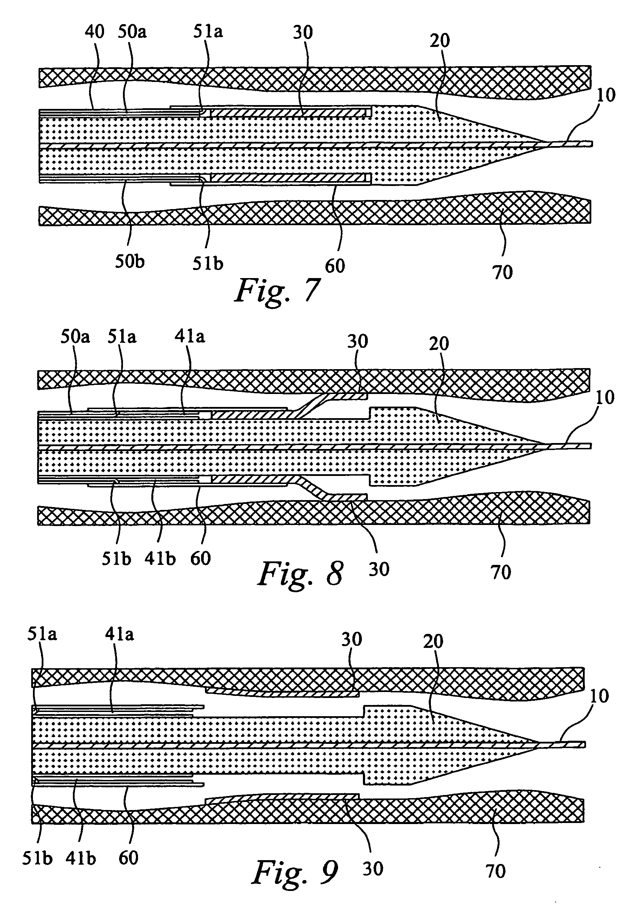

Figure 7 displays a longitudinal cross-sectional view through the introducer of figures 1, 2 and 3 when placed at a deployment location within bodily lumens (70), the section being taken through both pulling wire lumens (41 a, 41 b) and the central lumen (42). As may be seen from Figure 7 the distal ends of the pulling wires (51 a, 51 b) are attached to the radially interior surface of the outer sheath (60).

Figure 8 displays the introducer of Figure 7 with the pulling wires (50a, 50b) having been pulled a short distance proximally. This causes the ends of the pulling wires (51 a, 51 b) to tear slits in the surface of inner sheath (40), thus allowing the retraction of outer sheath (60) over inner sheath (40). A distal portion of the self-expandable stent (30) has been unsheathed and contacts the vessel walls (70).

Figure 9 displays the introducer of Figures 7 and 8 with the outer sheath (60) fully retracted. A radiopaque marker may advantageously be integrated in the distal end of the outer sheath (60) so as to enable the surgeon to visualise when the outer sheath (60) has been retracted sufficiently far to release the stent (30).

It will of course be appreciated by those skilled in the art that the foregoing description is exemplary only and that there will be a variety of ways of providing regions of weakness within the inner sheath. The use of grooves and different materials should only been seen as an example of the general principle of providing constructions, configurations or materials which provide a portion which is less resilient to tearing. For example, the use of grooves should be seen as equivalent to the use of perforation or pre-formed structural weakness of any kind.

The invention may be employed with catheter structures adapted for rapid exchange wire guide usage or with catheter structures having no wire guide lumen and adapted for insertion within a previously deployed introducer sheath.

Further, as a person skilled in the art will readily appreciate, the above description is meant as an illustration of the implementation of the principle of this invention. This description is not intended to limit the scope or application of this invention as defined in the following claims.

## Claims

1. Apparatus (100) for delivering an expandable prosthe (30) within a body lumen, comprising a catheter structure having at its distal end a prosthesis bearing site (23), a delivery sheath (60) positioned over said prosthesis bearing site so as to retain a prosthesis as the catheter structure is advanced through the body lumen, the delivery sheath being retractable to release the prosthesis; a pull wire lumen (41a, 41b) extending at least part way through the catheter structure from the proximal end of the delivery sheath; and a pull wire (50a, 50b) connected with the delivery sheath and extending through the pull wire lumen to enable remote retraction of the delivery sheath, wherein the distal end of the pull wire lumen is associated with a region of weakness in the catheter structure such that the region of weakness is torn on retraction of the delivery sheath, **characterized in that** said catheter structure comprises an inner sheath (40), said pull wire lumen and said associated region of weakness being located in the inner sheath.

2. Apparatus according to Claim 1, wherein said apparatus further comprises a barb (81a, 81b) that, on retraction of the delivery sheath, aids in tearing said region of weakness.

3. Apparatus according to Claim 2, wherein said barb is formed on an annular member (80) located around said catheter structure.

4. Apparatus according to Claim 2, wherein said barb is formed on said delivery sheath.

5. Apparatus according to any one of Claims 2 to 4, comprising a plurality of said barbs spaced equiangularly about said catheter structure.

6. Apparatus according to any one of the preceding claims, comprising a plurality of said pull wires spaced equiangularly about said catheter structure.

7. Apparatus according to any preceding claim, wherein one or more regions of weakness are provided at least in part by one or more grooves (43a, 43b) formed in said catheter structure.

8. Apparatus according to Claim 1, wherein said inner sheath consists substantially of a first material and said region of weaknes is provided at least in part by an elongate region of a second material.

9. Apparatus according to Claim 8, wherein said first material has greater shear strength than said second material.

10. Apparatus according to Claim 8, wherein said first material has greater scratch hardness than said second material.

11. Apparatus according to Claim 8, wherein said second material is anisotropic.

12. Apparatus according to any preceding claim, further comprising a selfexpandable stent disposed at said prosthesis bearing site.

## Patentansprüche

1. Gerät (100) zur Abgabe einer expandierbaren Prothese (30) in einem Körperlumen, das eine Katheterkonstruktion mit einer Prothesenauflagefläche (23) an ihrem distalen Ende, eine über der Prothesenauflagefläche angeordnete Abgabeschleuse (60) zum Halten einer Prothese, während die Katheterkonstruktion durch das Körperlumen vorgeschoben wird, wobei die Abgabeschleuse zur Freisetzung der Prothese zurückgezogen werden kann; ein sich zumindest teilweise durch die Katheterkonstruktion vom proximalen Ende der Abgabeschleuse erstreckendes Zugdrahtlumen (41a, 41b); und einen mit der Abgabeschleuse verbundenen und sich durch das Zugdrahtlumen erstreckenden Zugdraht (50a, 50b), um ferngesteuertes Zurückziehen der Abgabeschleuse zu gestatten, umfasst, worin das distale Ende des Zugdrahtlumens mit einer Schwächeregion in der Katheterkonstruktion assoziiert ist, so dass die Schwächeregion beim Zurückziehen der Abgabeschleuse zerrissen wird, **dadurch gekennzeichnet, dass** die Katheterkonstruktion einen Innenschleuse (40) umfasst, wobei das Zugdrahtlumen und die assoziierte Schwächeregion in der Innenschleuse angeordnet sind.

2. Gerät nach Anspruch 1, worin das Gerät ferner einen Widerhaken (81a, 81b) umfasst, der beim Zurückziehen der Abgabeschleuse das Zerreißen der Schwächeregion unterstützt.

3. Gerät nach Anspruch 2, worin der Widerhaken auf einem ringförmigen Glied (80), das um die Katheterkonstruktion angeordnet ist, geformt ist.

4. Gerät nach Anspruch 2, worin der Widerhaken auf der Abgabeschleuse geformt ist.

5. Gerät nach einem der Ansprüche 2 bis 4, das eine Vielzahl der im gleichwinkligen Abstand um die Katheterkonstruktion angeordneten Widerhaken umfasst.

6. Gerät nach einem der vorhergehenden Ansprüche, das eine Vielzahl von im gleichwinkligen Abstand um die Katheterkonstruktion angeordneten Zugdrähten umfasst.

7. Gerät nach einem der vorhergehenden Ansprüche, worin ein oder mehr Schwächeregionen zumindest teilweise von ein oder mehr in der Katheterkonstruktion geformten Kerben (43a, 43b) bereitgestellt werden.

8. Gerät nach Anspruch 1, worin die Innenschleuse im Wesentlichen aus einem ersten Material besteht und die Schwächeregion zumindest teilweise von einer länglichen Region aus einem zweiten Material bereitgestellt wird.

9. Gerät nach Anspruch 8, worin das erste Material eine größere Scherfestigkeit aufweist als das zweite Material.

10. Gerät nach Anspruch 8, worin das erste Material eine größere Kratzfestigkeit aufweist als das zweite Material.

11. Gerät nach Anspruch 8, worin das zweite Material anisotrop ist.

12. Gerät nach einem der vorhergehenden Ansprüche, das ferner einen selbstexpandierbaren Stent umfasst, der an der Prothesenauflagefläche angeordnet ist.

## Revendications

1. Appareil (100) permettant d'appliquer une prothèse (30) extensible à l'intérieur d'une lumière du corps, comportant une structure de cathéter présentant à son extrémité distale un site (23) d'appui de prothèse, une gaine (60) d'application positionnée sur ledit site d'appui de prothèse de façon à retenir une prothèse au fur et à mesure que la structure de cathéter est avancée dans la lumière du corps, la gaine d'application étant rétractable pour libérer la prothèse ; une lumière (41a, 41b) de fil de traction s'étendant à travers au moins une partie de la structure de cathéter depuis l'extrémité proximale de la gaine d'application ; et un fil (50a, 50b) de traction relié à la gaine d'application et s'étendant à travers la lumière de fil de traction afin de permettre la rétraction à distance de la gaine d'application, dans lequel l'extrémité distale de la lumière de fil de traction est associée à une zone de faiblesse dans la structure de cathéter de sorte que la zone de faiblesse est déchirée au moment de la rétraction de la gaine d'application, **caractérisé en ce que** ladite structure de cathéter comporte une gaine (40) interne, ladite lumière de fil de traction et ladite zone associée de faiblesse étant situées dans la gaine interne.

2. Appareil selon la revendication 1, dans lequel ledit appareil comporte en outre un cran (81a, 81b) qui, au moment de la rétraction de la gaine d'application, aide à déchirer ladite zone de faiblesse.

3. Appareil selon la revendication 2, dans lequel ledit cran est formé sur un organe (80) annulaire situé autour de ladite structure de cathéter.

4. Appareil selon la revendication 2, dans lequel ledit cran est formé sur ladite gaine d'application.

5. Appareil selon l'une quelconque des revendications 2 à 4, comportant une pluralité desdits crans espacés équiangulairement autour de ladite structure de cathéter.

6. Appareil selon l'une quelconque des revendications précédentes, comportant une pluralité desdits fils de traction espacés équiangulairement autour de ladite structure de cathéter.

7. Appareil selon l'une quelconque des revendications précédentes, dans lequel une ou plusieurs zones de faiblesse sont fournies au moins en partie par une ou plusieurs rainures (43a, 43b) formées dans ladite structure de cathéter.

8. Appareil selon la revendication 1, dans lequel ladite gaine interne est constituée sensiblement d'un premier matériau et ladite zone de faiblesse est fournie au moins en partie par une zone allongée d'un second matériau.

9. Appareil selon la revendication 8, dans lequel ledit premier matériau a une résistance au cisaillement plus grande que ledit deuxième matériau.

10. Appareil selon la revendication 8, dans lequel ledit premier matériau a une résistance à la rayure plus grande que ledit deuxième matériau.

11. Appareil selon la revendication 8, dans lequel ledit second matériau est anisotrope.

12. Appareil selon l'une quelconque des revendications précédentes, comportant en outre un stent auto extensible disposé au niveau dudit site d'appui de la prothèse.
